# EUROPEAN PATENT APPLICATION

(11) **EP 1 792 537 A1**
(43) Date of publication of application: **06.06.2007**
(21) Application number: 05768801.2
(22) Date of filing: 01.08.2005
(51) Int. Cl.: A01K 67/027, A61K 35/48, A61P 7/02, A61P 37/00, A61P 43/00, C12N 15/13, C12N 5/10, C12P 1/00

(54) **METHOD OF CONSTRUCTING CLONE MAMMAL**

(30) Priority: 18.08.2004 JP 2004238836; 17.06.2005 JP 2005177998
(71) Applicant: Riken, Wako-shi, Saitama 351-0198 (JP)
(72) Inventor: WAKAO, Hiroshi, RIKEN Yokohama Institute, Yokohama-shi, Kanagawa 230-0045 (JP); KOSEKI, Akihiko, RIKEN Yokohama Institute, Yokohama-shi, Kanagawa 230-0045 (JP); TANIGUCHI, Masaru, RIKEN Yokohama Institute, Yokohama-shi, Kanagawa 230-0045 (JP); OGURA, Atsuo, RIKEN Tsukuba Institute, Tsukuba-shi, Ibaraki 305-0074 (JP); INOUE, Kimiko, RIKEN Tsukuba Institute, Tsukuba-shi, Ibaraki 305-0074 (JP)
(74) Representative: Woods, Geoffrey Corlett
(86) International application number: PCT/JP2005/014474
(87) International publication number: WO 2006/018998

(57) **Abstract**

The present invention provides a method of producing a cloned mammal, which uses a mammalian natural killer T cell as a donor cell, a cloned mammal obtained by the method, a method of obtaining an ES cell from the embryo of the cloned animal and an ES cell obtained by the method.

## Description

### Technical Field

The present invention relates to a cloned mammal embryo and a cloned mammal, a method for producing the same, and use thereof and the like. The present invention also relates to cloned mammal embryonic stem cells (hereinafter also referred to as ES cells), a method for producing the same and use thereof and the like.

### Background Art

Since the birth of the cloned sheep Dolly in the UK (Wilmut, I. et al., "Viable offspring derived from fetal and adult mammalian cells", Nature (UK), 1997, 385, p.810-813), animal clones have been established by a variety of techniques, but the technique comprising transplanting a somatic cell-derived nucleus directly to an enucleated oocyte to obtain a clone is very poor in efficiency, and because it is extremely difficult to identify the origin of the donor cell used for the transplantation, there has been a problem with reproducibility. To overcome the latter, lymphocytes expressing a cell specific surface marker were used as donors, but the efficiency was poor and an extraordinary process was necessitated. For example, when peripheral lymphocytes such as T cells and B cells were used, ES cells were established only at a probability of about 1/500 (0.2%) (Hochedlinger, K., and R. Jaenisch., "Monoclonal mice generated by nuclear transfer from mature B and T donor cells", Nature, (UK) 2002, 415, p.1035-1038). Also, when olfactory sensory neurons are used as donors, the probability of the establishment of ES cells increases to some extent, but all these cases require the two steps of ES cell establishment and tetraploid complementation for the production of cloned individuals (Eggan, K. et al., "Mice cloned from olfactory sensory neurons", Nature (UK), 2004, 428, p.44-49). As stated above, preparing a clone using terminally differentiated peripheral cells has taken very much time and labor. On the other hand, considering applications to humans, because once converting to ES cells means that the clone's HLA does not match that of the donor (except for autologous transplantation), immunorejection is unavoidable so that the clone is unusable. Accordingly, there has been a demand for the identification of somatic cells that can be used as donor cells overcoming these technical and immunological limitations. Also, by conventional somatic cell clone technology, it is nearly impossible to accurately identify the origin of donor cells; it has been impossible to sorting out a group of cells having pluripotency for the whole body.

Also, by conventionally performed clone technology, only an extremely low number of clones actually bear babies after embryo transplantation; there is a demand for a method for preparing a cloned mammal that is more likely to bear babies.

### Disclosure of the Invention

It is an object of the present invention to provide a method for more efficiently preparing a cloned animal of higher survival rate, a cloned animal obtained by the method, offspring, embryo and the like thereof, and a cell, tissue, organ, and product obtained therefrom, and the like. It is another object of the present invention is to provide a method for obtaining ES cells from a cloned animal embryo, cloned animal ES cells obtained by the method, and a cell, tissue, organ, and product obtained therefrom, and the like. It is still another object of the present invention to provide a somatic cell clone technology enabling the accurate identification of the origin of donor cells.

The present inventors diligently investigated in view of the above-described problems, and as a result, found that a cloned embryo obtained by using a natural killer T cell (hereinafter also referred to as NKT cell) as the donor cell that provides a nucleus will show development and produce babies with no need of the establishment of ES cells and sequential tetraploid complementation. Furthermore, the present inventors confirmed that the cloned animal obtained had the capability of normal reproduction, and completed the present invention. Accordingly, the present invention is as follows:
[1] A method for preparing a cloned non-human mammal, which comprises using a mammalian natural killer T cell as the donor cell.
[2] A method for preparing a cloned non-human mammal, which comprises introducing the nucleus of a mammalian natural killer T cell into an enucleated mammalian oocyte.
[3] A method for preparing a cloned non-human mammal, which comprises introducing a nucleus derived from a mammalian natural killer T cell into an enucleated mammalian oocyte to form a reconstructed embryo, and transferring the reconstructed embryo into a host mammal.
[4] The method for preparing a cloned non-human mammal described in any of [1] to [3] above, wherein the natural killer T cell has been genetically manipulated to express a desired character.
[5] The method for preparing a cloned non-human mammal described in any of [1] to [4] above, wherein the natural killer T cell and the enucleated oocyte are derived from the same species of mammal.
[6] The method for preparing a cloned non-human mammal described in any of [1] to [4] above, wherein the natural killer T cell and the enucleated oocyte are derived from different species of mammals.
[7] The method for preparing a cloned non-human mammal described in any of [1] to [6] above, wherein the natural killer T cell is collected from a tissue selected from the group consisting of cord blood, peripheral blood, liver, bone marrow, spleen, and thymus.
[8] A cloned non-human mammal prepared by the method described in any of [1] to [7] above.
[9] A fetus of a cloned non-human mammal obtained by the method described in any of [1] to [7] above.
[10] An offspring of the cloned non-human mammal described in [8] above.
[11] The offspring of cloned non-human mammal described in [10] above, wherein the TCRVβ chain expressed on T cells is constituted by a substantially single TCRVβ chain repertoire.
[12] The offspring of cloned non-human mammal, described in [11] above, wherein the single TCRVβ chain repertoire is identical to the TCRVβ chain repertoire of the natural killer T cell used as the donor cell.
[13] The offspring of cloned non-human mammal described in [10] above, which has an allele comprising V_{α}14-J_{α}281, which is a TCR_{α} chain after gene rearrangement, wherein the number of natural killer T cells has been increased.
[14] A cell, tissue, organ or product obtained from the cloned non-human mammal described in [8] above.
[15] A cell, tissue, organ or product obtained from the fetus of a cloned non-human mammal described in [9] above.
[16] A cell, tissue, organ or product obtained from the offspring of cloned non-human mammal described in [10] above.
[17] The cell, tissue, organ or product described in [14] or [15] above, which is immunologically identical to the donor cell.
[18] The cell, tissue, organ or product described in any of [14] to [17] above, which is used for a treatment, an organ transplantation and/or a cell transplantation.
[19] A method for preparing a cloned mammal embryo, which comprises using a mammalian natural killer T cell as a donor cell.
[20] A method for preparing a cloned mammal embryo, which comprises introducing the nucleus of a mammalian natural killer T cell into an enucleated mammalian oocyte.
[21] The method for preparing a cloned mammal embryo described in [19] or [20] above, wherein the natural killer T cell has been genetically manipulated to express a desired character.
[22] The method for preparing a cloned mammal embryo described in any of [19] to [21] above, wherein the natural killer T cell and the enucleated oocyte are derived from the same species of mammal.
[23] The method for preparing a cloned mammal embryo described in any of [19] to [21] above, wherein the natural killer T cell and the enucleated oocyte are derived from different species of mammals.
[24] The method for preparing a cloned mammal embryo described in any of [19] to [23] above, wherein the natural killer T cell is collected from a tissue selected from the group consisting of cord blood, peripheral blood, liver, bone marrow, spleen, and thymus.
[25] A cloned mammal embryo prepared by the method described in any of [19] to [24] above.
[26] A method for preparing a cloned mammal embryonic stem cell, which comprises culturing the cloned mammal embryo described in [25] above until the blastocyst stage or the pre-blastocyst stage, and separating an embryonic stem cell from the obtained inner cell mass in the blastocyst stage or the pre-blastocyst stage.
[27] A cloned mammal embryonic stem cell obtained by the method described in [26] above.
[28] A method for preparing a differentiated cell, tissue, organ or product of a cloned mammal, which comprises culturing the cloned mammal embryonic stem cell described in [27] above under conditions allowing the induction of desired cell differentiation to induce the differentiation.
[29] A differentiated cell, tissue, organ or product of cloned mammal obtained by the method described in [28] above.
[30] The differentiated cell, tissue, organ or product described in [29] above, which is immunologically identical to the donor cell.
[31] The differentiated cell, tissue, organ or product described in [29] or [30] above, which is used for a treatment, an organ transplantation and/or a cell transplantation.

### Brief Description of the Drawings

Fig. 1 is a schematic diagram showing an outline of the preparation of an NKT cell-derived cloned mouse and the establishment of an ES cell line.
Fig. 2 is a schematic diagram showing the positions of the TCRV_{α} chain gene loci and the primers.
Fig. 3 is a schematic diagram showing the positions of the TCRVβ chain gene loci and the primers.
Fig. 4 is a drawing showing the results of a Southern blot analysis using genomic DNAs from NKT cloned mouse #1 (tail and placenta), #2 (tail and placenta), #3 (placenta), and #4 (dead birth, placenta) (electrophoretic photographs). The open arrowheads indicate bands after occurrence of gene rearrangement, and the closed arrows indicate bands prior to occurrence of gene rearrangement (germline configuration).
Fig. 5 is a drawing demonstrating a TCRV_{α}14-J_{α}281 gene rearrangement in cloned mice using a PCR method (electrophoretic photograph).
Fig. 6 is a drawing showing the TCRV_{α} chain base sequences in NKT cell-derived cloned mice.
Fig. 7 is a drawing showing the TCRVβ chain base sequences in NKT cell-derived cloned mice.

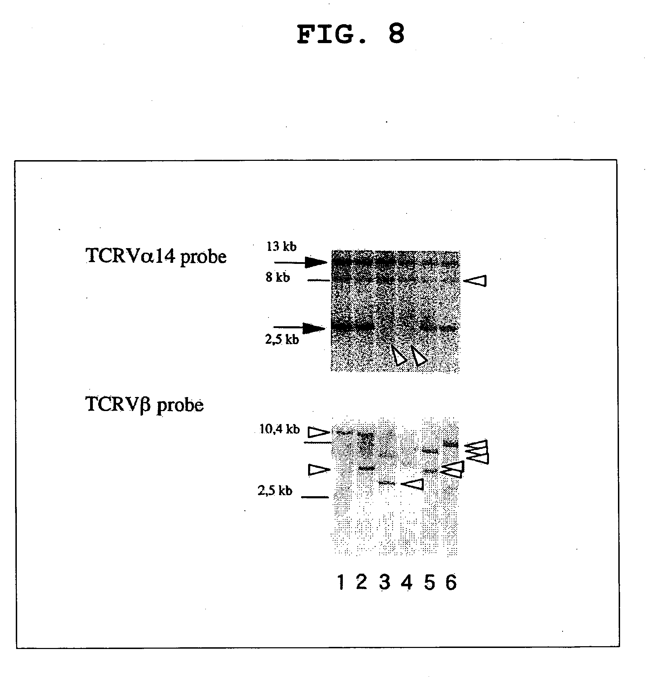

Fig. 8 is a drawing showing the results of a Southern blot analysis using NKT cell-derived ES cell (lanes 1 to 6) genomic DNAs (electrophoretic photographs). The probes were the same as those used for the cloned mouse analysis. With the TCRV_{α}14 probe, a shift of the 13-kb band was observed (lanes 1 to 6), and in the ES cells on lanes 3 and 4, disappearance of the 2.5-kb band was observed. Also, when the TCRV_{β} probe was used as well, bands after completion of gene rearrangement were observed. The open arrowheads indicate bands after occurrence of gene rearrangement. The closed arrows indicate bands prior to occurrence of gene rearrangement (germline configuration).

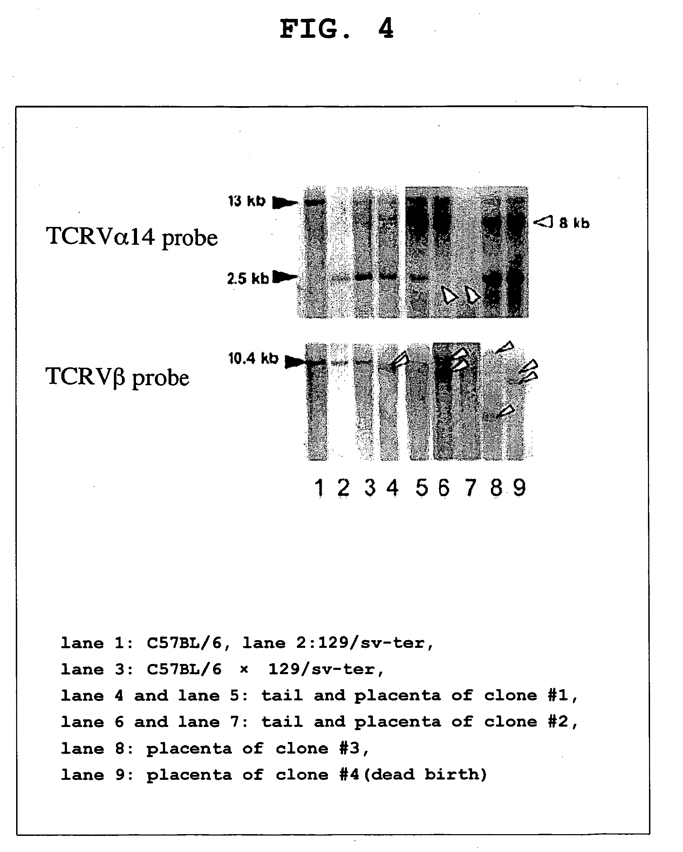

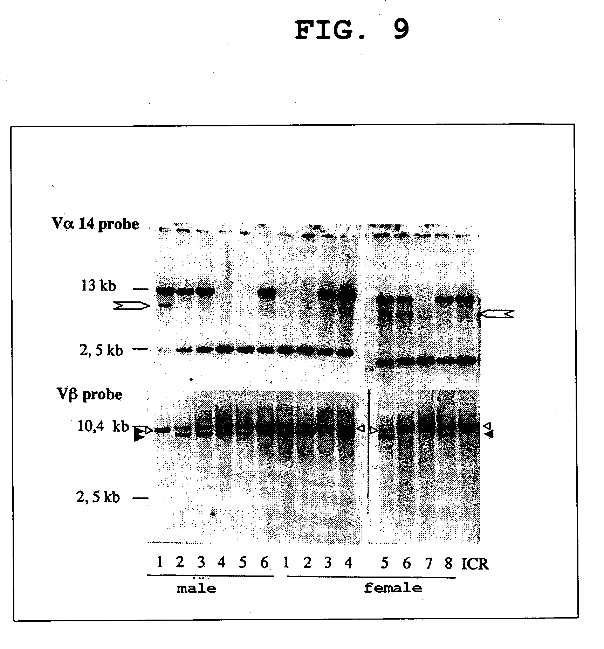

Fig. 9 is a drawing showing the results of a Southern blot analysis in F1 animals obtained by crossing NKT cloned mouse #1 and an ICR mouse (electrophoretic photographs). F1 animals inheriting an 8-kb band derived from TCRV_{α}14 after gene rearrangement derived from a cloned mouse are seen in male No. 1 and female No. 6 (upper panel, arrows). Also, alleles comprising TCRVβ derived from cloned mouse #1 are segregated at about 9 kb and 8 kb, respectively (see Fig. 4, closed arrowheads). Each F1 animal has inherited a 10.4-kb band derived from the mother mouse (open arrowhead) and about 9-kb or 8-kb band.

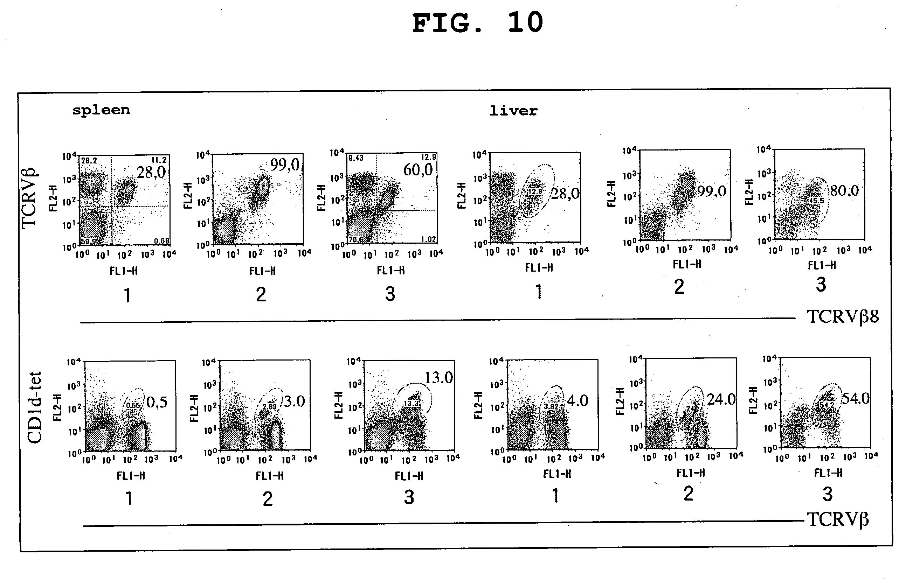

Fig. 10 is a drawing showing the results of a FACS analysis in F1 animals obtained by crossing NKT cloned mouse #1 and an ICR mouse. The upper panel shows the ratio of TCRVβ8 in the cloned mouse offspring as the ratio to all TCRVβ-positive cells. The lower panel shows the ratio of NKT cells in the cloned mouse offspring. Panel 1, panel 2, and panel 3 show the results from a control ICR mouse (wild-type), from a mouse genetically inheriting in-frame TCRVβ8 after completion of gene rearrangement, and from a mouse inheriting V_{α}14-J_{α}281 after completion of a gene rearrangement thereof, respectively. Each cluster of NKT cells is encircled, and the number of NKT cells to the total number of cells is shown as percent (%).
Fig. 11 is a drawing showing the results of an analysis of the differentiation or survival status of reconstructed embryos obtained by transplanting an NKT cell nucleus or T cell nucleus, in in vitro culture (48 and 72 hours), in graphic representation of the results obtained at 48 and 72 hours in Table 1 (excluding the birth of mice). The ordinate indicates changes over time in the survival rate of cultured embryo, or the incidence of individuals obtained by introducing a reconstructed embryo into a pseudo-pregnant mouse (birth rate of transplanted embryo).
   *P<0.0001; **p<1×10⁻²⁵
Fig. 12 is a drawing showing the results of a FACS analysis of peripheral lymphocytes from cloned mice (#1, #2) (TCRVβ vs TCRVβ8).
Fig. 13 shows the results of measurements by ELISA of levels of cytokines (IFN-γ, IL-2, GM-CSF, IL-4, IL-10, IL-5, TNF-α, IL-1β) in sera collected from mice after the elapse of 0, 4, 12, and 24 hours following stimulation with α-GC, which is an artificial agonist of NKT cells. At each measurement time, four mice were measured. The ordinate indicates the concentration of each cytokine, and the abscissa indicates measurement points. Data are expressed as mean ± standard deviation.
Fig. 14 shows the results of measurements of the amounts of IL-4 and IFN-γ in the culture supernatant of a mixed culture of dendritic cells (DC) (5 × 10⁴ cells), previously treated with α-GC, and splenocytes (SPC) as the responding cells (1 × 10⁶ cells). The ordinate indicates the concentration of each cytokine, and the abscissa indicates the origin of splenocytes. Data are expressed as mean ± standard deviation for three measurements.

### Detailed Description of the Invention

Unless otherwise specified in the sentences, all technical terms and scientific terms used in the present specification have the same meaning generally understood by those of ordinary skill in the art in the technical field to which the present invention belongs. Any method or material similar or equivalent to those described in the present specification can be used for the practice or experiment of the present invention. Preferable methods and materials are described in the following. Any publication and patent cited in the present specification are incorporated in the present specification for reference, with the aim of, for example, describing and disclosing constructs and methodologies described in publications, which can be used in relation to the invention described herein.

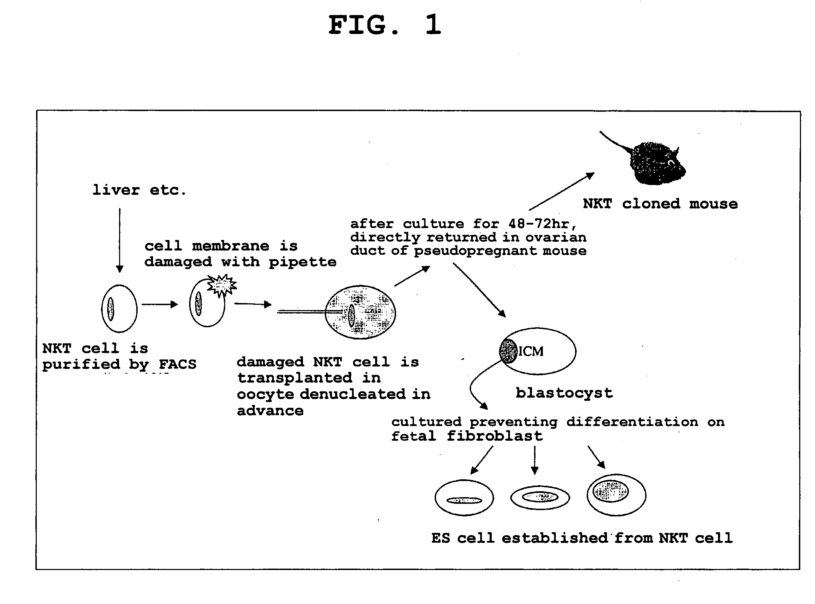

The present invention is explained in detail in the following. An outline of one example of the present invention is shown in Fig. 1.

Natural killer T (NKT) cell to be used in the present invention is one kind of lymphocytes having a regulatory role in the immune system, though its population is small. NKT cell has two antigen receptors; such as T cell receptor (TCR) and NK receptor. NKT cell expresses a specific repertoire different from conventional T cells and NK cells. For example, as for mouse β chain, not less than 90% of NKT cells mainly express limited repertoires of Vβ8, and additionally Vβ7 and Vβ2, and as for α chain, they express uniform Vα14-Jα281. The uniform TCRα chain is constructed as a result of selection of Vα14 gene and Jα281 gene from V and J gene groups and rearrangement thereof on the genome, during rearrangement of the TCR gene (Taniguchi et al., (2003) Annu Rev Immunol 21, 483-513 The regulatory role of Valpha14 NKT cells in innate and acquired immune response). In human, the combination is known to be of a non-polymorphic Vα24 having high homology with mouse Vα14, and Vβ11 analogous to Vβ8.2. Such property is suitable for the pursuit of the origin of donor cell in the obtained cloned animal. The origin of NKT cell is not particularly limited and the cell can be recovered from the cord blood, peripheral blood, liver, bone marrow, spleen, lymph node, thymus and the like of mammals such as primates including human, rodents, rabbit, cat, dog, horse, bovine, sheep, goat, swine and the like. The term "primate" used in the present specification includes, but is not limited to, any animal belonging to the group of mammals including monkey, ape and human. Specifically, NKT cells can be selected and recovered by FACS analysis of a suspension of a single cell recovered from peripheral blood or a homogenate of the liver, using a glycolipid antigen such as α-galactosylceramide (α-GalCer or α-GC) bound to CD1d molecule that can be recognized by a TCR highly retained in NKT cells. NKT cell used in the present invention may or may not be activated. A cell not stimulated by antigen is preferable. Alternatively, it may be an NKT cell obtained by culturing an NKT precursor cell in the presence of a factor that confers NKT cell differentiation induction ability. NKT precursor cell may be derived from fetal hepatocyte or peripheral blood or cord blood.

A method for preparation of a cloned mammal of the present invention is characterized in that an NKT cell is used as a donor cell. Specifically, the method is performed by nuclear transplantation of NKT cell. The method of nuclear transplantation is not particularly limited, as long as the nucleus of NKT cell can be used as a donor cell nucleus. It includes introduction of a cell nucleus into an enucleated oocyte derived from the same species of mammal as the cell nucleus and introduction of a cell nucleus into an enucleated oocyte derived from a different mammal species from the cell nucleus. The technique of nuclear transplantation into an enucleated oocyte derived from a different species of mammal is useful for the restoration of an extinct species and preservation and growth of species at the risk of extinction. Nuclear transplantation into an enucleated oocyte derived from the same species of mammal leads to an offspring more efficiently.

The method for introducing the nucleus of NKT cell into a mammalian enucleated oocyte is not particularly limited as long as the nucleus of an unfertilized egg can be finally replaced by the nucleus of a donor NKT cell. In consideration of the size of the NKT cell, a method comprising scratching a cellular membrane of NKT cell with a pipette etc., and introducing the scratched NKT cell directly into an enucleated oocyte using a micromanipulator and the like is preferably used. Nuclear transplantation may be performed by cell fusion of NKT cell and enucleated oocyte. More specifically, for example, nuclear transplantation can be performed according to the methods reported by Wakayama et al. (Nature 394, 369-374 (1998)) and Inoue et al. (Biol. Reprod. 69, 1394-1400 (2003)), or performed after appropriate modification. When nuclear transplantation is to be performed by cell fusion of NKT cell and enucleated oocyte, electrofusion using a commercially available apparatus can be employed.

A mammalian oocyte to be enucleated can be obtained by an ovarian hyperstimulation treatment by hormone administration. Enucleation is performed by sucking the nucleus and surrounding cytoplasm of an unfertilized oocyte into a small pipette, and tearing them off. However, by this operation, the cytoskeleton is broken and the oocyte is destroyed. Accordingly, a treatment to eliminate the cytoskeleton of an unfertilized oocyte is preferably performed in advance. The cytoskeleton can be eliminated by the use of cytochalasin. In addition, it is preferable to apply a treatment with cytochalasin etc. after the nuclear transplantation. The oocyte to be subjected to nuclear transplantation may or may not be activated, but an activated state is preferable. The activation can be applied before or after the nuclear transplantation. While activation can be performed by, but is not particularly limited to, stimulation with an electric method or a drug treatment. Particularly preferably, naturally matured oocyte in the telophase is used. The activation can be performed, for example, by increasing the concentration of divalent cation in the oocyte, and/or lowering the level of phosphorylation of cellular protein in the oocyte. This is generally performed by introducing a divalent cation, such as magnesium, strontium, barium and calcium, into the cytoplasm of oocyte, for example, in the form of an ionophore. Other methods for increasing a divalent cation concentration include use of an electric shock, an ethanol treatment and a treatment with a caged chelating agent. The level of phosphorylation may sometimes be lowered by a known method, for example, the addition of a kinase inhibitor (e.g., serine-treonine kinase inhibitors such as 6-dimethylaminopurine, 2-aminopurine and sphingosine). Alternatively, phosphorylation may also be inhibited by introduction of phosphatase into the oocyte.

A cloned mammal can be obtained by introducing the nucleus of NKT cell into an enucleated oocyte to reconstruct an embryo, and transplanting the reconstructed embryo into a host mammal to allow the host mammal to develop the reconstructed embryo.

As mentioned above, when a NKT cell is used as a donor cell, a cloned mammal can be obtained by somatic cell nuclear transplantation without the need of establishment of ES cell and sequential tetraploid embryo complementation. In other words, NKT cell is assumed to have totipotency.

The expression "have totipotency" as used in the present specification means a cell that produces any cell in a cell mass under development, such as embryo, fetus, animal and the like. In a preferable embodiment, "have totipotency" means a cell that produces any cell in an animal. A cell having totipotency can produce any cell in a cell mass under development when used in the procedure employed for producing embryo from one or plural nuclear transplantation steps.

The expression "have totipotency" as used in the present specification is distinguished from the expression "have pluripotency". The latter term means a cell that can differentiate into a cell subpopulation in a cell mass during development, but cannot produce all cells in the cell mass during development.

The terms "embryo" and "embryonic" used in the present specification include a cell mass during development, which has not been implanted in the uterus membrane of the host mammal. Therefore, the terms "embryo" and "embryonic" used in the present specification may mean fertilized oocyte, cytoplasmic hybrid, cell mass during development in the pre-blastocyst stage and/or any other cell mass during development, which is in the developmental stage before implantation in the uterus membrane of a host mammal. A reconstructed embryo means an embryo reconstructed from the nucleus derived from a donor cell and the cytoplasm component derived from an oocyte, which is obtained by nuclear transplantation of the donor cell nucleus in an enucleated oocyte.

The embryo may show multiple stages of cell development. For example, one cell embryo can be called a zygote, the zygote is a solid and spherical cell mass developed from a split embryo, which can be called a morula, and an embryo having a blastocele can be called a blastocyst.

The transplantation of a reconstructed embryo into a host mammal can be performed according to a method generally employed in the pertinent field. To be specific, a cloned mammal is produced by continuously culturing a reconstructed embryo up to the 2 to 8-cell stages, transplanting the embryo of the 2 to 8-cell stages to a host mammal, and allowing the mammal to develop the reconstructed embryo. A medium suitable for culture and maturation of the reconstructed embryo are technically well known, and are appropriately set according to the kind of mammal to be the origin of the enucleated oocyte. The reconstructed embryo can be cultured together with a feeder cell, preferably cultured on a feeder cell layer when desired. The reconstructed embryo is cultured up to the size suitable for transplantation in a host mammal. The reconstructed embryo may be cultured to reach preferably about 2 - 400 cells, more preferably about 4 - 128 cells (in the case of a mouse, about 48 hr - about 72 hr). The culture is performed under suitable conditions, namely, at about 37°C - 38.5°C and in the presence of about 5 - 7.5% CO₂, and the medium is appropriately changed. The host mammal is not particularly limited as long as a reconstructed embryo can be developed therein, and is preferably a mammal derived from the same species as the enucleated oocyte, more preferably a pseudopregnant female mammal. In the case of a mouse, for example, a pseudopregnant female mouse can be obtained by crossing a female mouse with normal sexual cycle with a male mouse emasculated by vasoligation and the like. A reconstructed embryo (cloned embryo) obtained by the aforementioned method is transplanted in the uterus of the prepared pseudopregnant mouse, particularly in the ovarian duct, which is followed by pregnancy and delivery to produce a cloned mammal. To secure implantation and pregnancy of a cloned embryo, the pseudopregnant mouse to be a foster parent is preferably selected from a female mouse group having the same sexual cycle with a female mouse from which an oocyte to be enucleated is recovered.

The term "fetus" to be used in the present specification means a cell mass during development, which has been implanted in the uterus membrane of a host mammal. A fetus may show a clear feature of, for example, genital ridge. A genital ridge is a feature easily identified by those of ordinary skill in the art, and is recognizable in the fetuses of many animal species. The fetal cell may mean any cell isolated from and/or produced by a fetus, or a cell derived from a fetus. The fetus of the cloned mammal of the present invention can be obtained in any stage after transplantation of a reconstructed embryo in a foster parent during the above-mentioned production process of a cloned mammal.

An offspring of the cloned mammal of the present invention can be obtained by crossing a cloned mammal developed from a reconstructed embryo with a second mammal. The second mammal may be a normal mammal (not a clone) of the same species as the cloned mammal (to be also conveniently referred to as a first mammal), or a cloned mammal developed from a cloned embryo or an offspring thereof and the like. It may be a cloned mammal developed from the below-mentioned transgenic mammal or transgenic mammal embryo, or an offspring thereof. In the present invention, the "offspring" may be of any generation, such as a child generation (F1) from the cloned mammal of the present invention as a parent, a child generation (F2) from F1 as a parent, a child generation (F3) from F2 as a parent and the like, as long as it is originally developed from the cloned mammal of the present invention. In the present invention, any cell, tissue or organ of the above-mentioned cloned mammal, a fetus of the cloned mammal, or an offspring of the cloned mammal can be obtained. The cell, tissue and organ do not need to be harvested or recovered by any particularly limited method, and a method generally employed in the pertinent field can be employed. Moreover, any product secreted, produced or extracted from the obtained cell, tissue or organ is within the scope of the present invention. While a wide variety of products can be obtained from a cloned mammal, as in the case with the products obtained from the object mammal, glycoprotein, neuropeptide, immunoglobulin, enzyme, peptide and hormone can be mentioned. More specifically, human α1 antitrypsin, human blood coagulation factor VIII, tPA (tissue specific plasminogen activator), antithrombin III, protein C, fibrinogen, human blood coagulation factor IX and the like can be mentioned.

The tissue and organ of the cloned mammal obtained as mentioned above have the same histocompatibility antigen (e.g., HLA in human) as does the donor NKT cell, and when the tissue or organ obtained from the cloned mammal is transplanted in a mammal, the provider of the donor cell, it is not recognized as nonself, leading to immunological tolerance, and an immunological rejection does not occur.

Moreover, an offspring of the cloned mammal of the present invention has the following characteristics (detailed in the below-mentioned Examples).
(1) TCRVβ chain expressed in an individual has substantially a single TCRVβ chain repertoire, particularly the same TCRVβ chain repertoire as that of NKT cell used as the donor cell.
(2) The number of NKT cells has increased.

Rearrangement of TCR gene group plays an important role in the formation of a huge number of repertoire of lymphocytes involved in the differentiation and selection of T cells. For example, expression of recombination activating gene (RAG) is essential for gene rearrangement thereof, and it has been clarified that abnormal or deficient RAG causes severe combined immunodeficiency and omen syndrome.

A phenomenon as in the above-mentioned (1) wherein TCR in an individual is limited to particular one kind has not been reported except for TCR transgenic animals. Using an offspring of a cloned mammal having such characteristics, the differentiation and selection of only one kind of T cell clone, which is originally one out of 100000 cells or million cells, can be observed at an individual level. For example, in the T cell expressing a single TCRVβ chain, αβ TCR is expressed and the expression of γδ TCR is suppressed. This phenomenon is similar to the phenomenon observed in bowel diseases such as colitis and the like, and therefore, an offspring of the cloned mammal of the present invention is useful as a model animal of colitis and the like. Furthermore, in a cloned mammal's offspring of the present invention having an allele containing Vα14-Jα281, which is a TCRα chain after gene rearrangement, NKT cell increases in number. An increase in the number of NKT cells is also observed in the proportion (of NKT cells) relative to the whole spleen cells, as well as in the absolute number. Since NKT cell has a function to regulate the immune system, the offspring of the cloned mammal of the present invention having a larger number of NKT cells is expected to greatly contribute to the study of disease and/or pathology involving NKT cells, particularly elucidation of the onset mechanisms of autoimmune diseases and allergic diseases, rejection of transplanted bone marrow, and tumor immunity. More specific examples of diseases and/or pathologies include inflammation, various pains, collagen diseases, autoimmune diseases, various immune diseases, inflammation and pain particularly in joint and muscle (chronic articular rheumatism, rheumatoid spondylitis, osteoarthritis, gouty arthritis etc.), skin inflammation (eczema etc.), ophthalmic inflammation (conjunctivitis etc.), pulmonary disorder accompanying inflammation (asthma, bronchitis etc.), condition of digestive organs accompanying inflammation (aphthous ulcer, Crohn's disease, atrophic gastritis, verrucousgastritis, ulcerative colitis, steatorrhea, regional ileitis, irritable bowel syndrome etc.), gingivitis, (inflammation, pain and swelling after operation or disorder), fever, pain and other condition relating to inflammation, rejection of transplantation, systemic lupus erythematosus, scleroderma, polymyositis, polychondritis, periarteritis nodosa, ankylosing spondylarthritis; inflammatory chronic renal conditions (glomerulonephritis, lupus nephritis, membranous nephritis etc.), rheumatic fever, Sjogren's syndrome, Behcet's disease, thyreoiditis, Type I diabetes, dermatomyositis, chronic active hepatitis, myasthenia gravis, graves disease, multiple sclerosis, primary biliary cirrhosis, autoimmune blood diseases (hemolytic anemia, pure red cell anemia, idiopathic thrombocythemia, aplastic anemia etc.), uveitis, contact dermatitis, psoriasis, Kawasaki's disease, disease involving Type I allergic response (allergic asthma, atopic dermatitis, urticaria, allergic conjunctivitis, pollinosis etc.), shock (septic shock, anaphylatic shock, adult respiratory distress syndrome etc.), sarcoidosis, Wegener's granuloma, Hodgkin's disease, cancer (lung cancer, gastric cancer, colon cancer, gastric cancer, hepatic cancer etc.), virus disease (hepatitis) and the like in human and animals.

The present invention also provides a production method of a cloned mammalian ES cell, which uses the above-mentioned reconstructed embryo (also referred to as cloned embryo or cloned mammalian embryo) and a cloned mammalian ES cell obtained thereby. ES cell includes a cell having pluripotency, which is preferably isolated from an embryo maintained by in vitro cell culture. While ES cell can be cultured irrespective of the presence of a feeder cell, a feeder cell is preferably used. As the feeder cell, those generally employed in the pertinent field can be used, in the case of a mouse ES cell, for example, a fibroblast derived from a mouse fetus can be used. A cloned mammalian ES cell can be established from an embryonic cell isolated from an embryo in any developmental stage, inclusive of an embryo in the blastocyst stage and an embryo in the pre-blastocyst stage. More specifically, it can be produced by introducing the nucleus of a donor NKT cell into an enucleated oocyte, culturing the obtained cloned mammalian embryo up to blastocyst stage and/or pre-blastocyst stage, and isolating from the obtained inner cell mass. The term "inner cell mass" used in the present specification means a cell that gives rise to an embryo itself. The cell flanking the outside of blastocyst is called an embryonic trophoblast. A method for isolating an inner cell mass from an embryo is known to those of ordinary skill in the art.

The cloned mammalian ES cell can be cultured to induce differentiation under the conditions capable of production of desired cell differentiation, and a desired differentiated cell, tissue or organ. The conditions under which a desired cell differentiation can be induced should be determined according to the kind and level of differentiation, which can be easily set by those of ordinary skill in the art. For example, embryonic stem cells are induced to differentiate into hematopoietic stem cells, and further differentiated to finally give blood cells such as erythrocytes, leukocytes and the like. Alternatively, they can be differentiated to neural stem cells and induced to differentiate into individual nerve cells.

Whether or not the fetus or baby obtained in the present invention is derived from a donor NKT cell can be confirmed by examining the gene of the obtained fetus or baby, specifically by examining the TCR gene. The TCR gene is rearranged in NKT cell and, as a result, the α chain characteristically expresses Vα14-Jα281 as mentioned above, for example, in mouse. Furthermore, it has a Vβ chain after gene rearrangement. The genomic DNA of fetus or baby of a cloned mammal that inherited the genetic information of NKT cell is subjected to Southern blot analysis using a TCRVα14 probe and/or a TCRVβ probe, preferably both a TCRVα14 probe and a TCRVβ probe, and whether the TCR gene is rearranged, particularly whether the TCR gene shows the same TCR rearrangement pattern as that of the NKT cell used as a donor cell, is confirmed, based on which the fetus or baby of the obtained cloned mammal is derived from the NKT cell, which is a donor somatic cell, can be determined. In addition, whether or not the rearranged TCR gene is inherited can also be confirmed by PCR. Specific steps for the confirmation are mentioned below in Examples.

A method for producing the above-mentioned cloned mammal of the present invention can also be utilized for cloning a genetically engineered mammal or transgenic mammal. Specifically, the method is characterized in that an NKT cell of a mammal genetically engineered to express the desired trait is used as a donor cell. More particularly, the nucleus of an NKT cell of a mammal genetically engineered to express the desired trait is introduced into an enucleated oocyte to a reconstruct embryo (transgenic embryo), and the reconstructed embryo is transferred to a host mammal. The "transgenic embryo" means an embryo containing a heterologous nucleic acid into which one or multiple cells have been introduced by intervention of human. The transgene can be directly or indirectly introduced into a cell by introduction into a cell precursor, intentional gene manipulation, or infection with a recombinant virus. The transgenic embryo described in the present specification expresses a structural gene capable of showing desired characteristic of a cell due to the transgene. However, it also includes a transgenic embryo wherein the transgene is silent. The transgenic embryo is the same as the one obtained by the above-mentioned process for producing a cloned mammal except that the cell nucleus of a donor cell is that of a cell genetically engineered to express the desired characteristic, and can be produced in the same manner.

The transgenic mammal of the present invention includes germline transgenic animal conferred with an ability to transmit genetic information to an offspring by introduction of genetic alteration or genetic information into a germline cell.

The term "gene" means a DNA sequence containing a regulatory sequence and a coding sequence necessary for the production of polypeptide or precursor proteins. Polypeptide may be encoded by a full length coding sequence, or may be encoded by an optional part of a coding sequence, as long as the desired activity is maintained.

The term "transgene" broadly means, but is not limited to, any nucleic acid that can be introduced into an animal genome, and includes a gene or DNA having a sequence probably generally absent on a genome, a gene which is present but generally not transcribed or translated (expressed) by a given genome, or any other gene or DNA desired to be introduced into the genome. The transgene may include a gene generally present on a non-transgenic genome but desired to show altered expression, or a gene desired to be introduced as a modified or atypical gene. The transgene may be specifically directed to a limited gene locus, or intrachromosomally incorporated at random, or become extrachromosomally replicated DNA. The transgene may contain one or multiple transcription regulatory sequences, and any other nucleic acid such as intron etc., which may be necessary for appropriate expression of the selected nucleic acid. The transgene may be a coding sequence or a non-coding sequence, or a combination thereof. The transgene may contain a regulatory element having an ability to drive expression of one or multiple transgenes under appropriate conditions.

The expression "structural gene capable of showing desired characteristic" means, for example, a structural gene that expresses a protein or antisense RNA showing desired characteristic (e.g., showing biological activity). The structural gene may be entirely or partially derived from any supply source known in the technical field, which contains genome or episome of plant, fungi, animal or bacterium, nucleus DNA or plasmid DNA of eucaryote, cDNA, virus DNA, or chemically synthesized DNA. The structural gene sequence may encode a polypeptide such as receptor, enzyme, cytokine, hormone, growth factor, immunoglobulin, cell cycle protein, intercellular signaling protein, membrane protein, cytoskeleton protein, or reporter protein (e.g., green fluorescence protein (GFP), β-galactosidase, luciferase) and the like. Further, the structural gene may be a gene relating to a particular disease or disorder such as cardiovascular disease, nervous disease, reproductive failure, cancer, ophthalmic disease, endocrine disorder, pulmonary disease, metabolic disorder, autoimmune disorder, senescence and the like.

The structural gene may contain, in a coding region or untranslated region, one or multiple modifications capable of affecting biological activity or chemical structure, expression rate, or expression regulatory mode of an expression product. Such modification includes, but is not limited to, one or multiple mutations, insertions, deletions and substitutions of nucleotide. The structural gene may consist of sequential coding sequence or contain one or multiple introns bound to appropriate splice junction. The structural gene may encode a fusion protein.

It is also possible to prepare an ES cell of a transgenic mammal using a transgenic embryonic cell, by a method according to the method for preparing an ES cell of the above-mentioned cloned mammal of the present invention. The obtained transgenic mammalian ES cell can be cultured and differentiation induced under conditions capable of inducing a desired cell differentiation, as in the aforementioned cloned mammalian ES cell, and can produce a desired differentiated cell, tissue or organ. The obtained differentiated cell, tissue or organ has a structural gene capable of showing a desired characteristic, and differentiated cell, tissue or organ having a desired characteristic or a product produced thereby can be obtained by expressing the gene. The cell, tissue or organ, or a product recovered therefrom can be recovered by a method generally employed in the pertinent field, and appropriately set according to the desired character. Moreover, it is also possible to produce an ES cell of a transgenic primate capable of expressing a gene relating to a particular disease, by the method explained in the present invention. Therefore, many human diseases can be treated by the method explained in the present invention, the obtained ES cell and the like.

The present invention provides, as mentioned above, a method for preparing a cloned mammal, as well as the possibility of preparing a transgenic cloned mammal. Such transgenic mammal can be used as a study model of severe human disease, or a model for evaluating the effect of treatment policy of gene or cell. The stem cell obtained by the present invention is extremely important for the research of many diseases and functions (e.g., senescence, AIDS, cancer, Alzheimer's disease, autoimmune abnormality, metabolic disorder, obesity, organ formation, mental diseases, and reproduction).

The TCRV_{β} chain of the cloned mammal and transgenic animal of the present invention is considered to be single and have a relatively or absolutely increased NKT cell number.

Using cloned animal and transgenic animal, the onset mechanism of a disease can be found and molecular medicinal treatment method can be constructed and optimized. For example, discovery of the treatment methods of cancer, arteriosclerosis causing cardiac disease and cerebral apoplexy, congenital metabolic abnormality, and other fetal disease and neonatal disease can be promoted using a mammal prepared by gene knockout of a particular gene. Such animal is also suitable for the evaluation and improvement of cell therapy of diseases including diabetes, hepatopathy, nephropathy, development of artificial organ, wound healing, damage due to heart attack, brain damage due to cerebral apoplexy, spinal injury, amnesia, Alzheimer's disease, as well as other dementia and injury of muscle and nerve.

### Examples

The present invention is explained in detail in the following by referring to Examples, which are not to be construed as limitative of the scope of the present invention. Any publication cited in the entirety of the present invention is incorporated into the present specification by reference. In addition, the reagents, apparatuses and materials used in the present invention are commercially available unless otherwise specified.

### Example 1: Preparation of cloned mouse derived from NKT cell

Mononuclear cells were acquired from the liver of 8-week-old to 23-week-old (C57BL/6x129/Sv-ter) F1 female mice by the Percoll (trade name, Amersham) specific gravity centrifugation method. The cells were stained with self-prepared Phycoerythrin (PE)-α-galactosylceramide (α-GC)-bound CD1d tetramer (α-GC loaded CD1d-tetramer; Matsuda, J.L., O.V. Naidenko, L. Gapin, T. Nakayama, M. Taniguchi, C.R. Wang, Y. Koezuka, and M. Kronenberg. 2000. Tracking the response of natural killer T cells to a glycolipid antigen using CD1d tetramers. J Exp Med 192:741-754.) and fluoroscein isothio cyanate (FITC)-anti-TCRVβ antibody (H57) (PharMingen), and the cells stained by them were used as NKT cells (i.e., NKT cells are defined by α-GC loaded CD1d-tetrame⁺/TCRβ⁺).

These cells were purified as PE⁺/FITC⁺ cell population by flow cytometer MoFlo (registered name) (Cytomation) and used as a donor for nuclear transplantation. Sorting was repeated twice to give NKT cells at a purity of not less than 99%.

The oocyte of the recipient was taken out by ovary flushing from a B6D2F1 female mouse treated with an ovulation inducing agent, and a cumulus cell was removed by a hyaluronidase treatment, enucleated with a Piezo-micromanipulator under an inverted microscope and used for nuclear transplantation. NKT cell was sucked into a pipette and the cellular membrane thereof was damaged by the physical stimulation with the Piezo-micromanipulator. Then, the zona pellucida and the cellular membrane of the enucleated oocyte were punctured by the Piezo-micromanipulator and the NKT cell nucleus was injected to the inside of the cytoplasm, whereby the nuclear transplantation was completed. Using the T cells in the same manner, the T cell nucleus was injected to the inside of the cytoplasm to give a reconstructed oocyte, which was used as a control.

After 1 hr from the nuclear transplantation, the reconstructed oocyte was activated in a KSOM medium containing cytochalasin and strontium for 1 hr, cultured in a KSOM medium containing cytochalasin alone for 5 hr, and in a KSOM medium, with an over time observation. After culture for 24 hr, most of the reconstructed embryos derived from NKT cell and T cell were in a 2-cell stage assumed to be the G0 stage. After additional 24 hr culture (total 48 hr), the reconstructed embryo obtained by transplantation of the nucleus derived from T cell remained in the 2-cell stage, but the reconstructed embryo obtained by transplantation of the nucleus derived from NKT cell developed to the 4-cell stage. By continuous culture (total 72 hr), 71% of the reconstructed embryo obtained by transplantation of the nucleus derived from NKT cell became morula·blastocysts, but the proportion in the reconstructed embryo obtained by transplantation of the nucleus derived from T cell was only 12%.

The cells were cultured for 48 hr to 72 hr (corresponding to 4-cell stage, morula-blastocyst, respectively), and returned to the ovarian duct of ICR mouse on day 1 of pseudopregnancy. After 19 days, the baby was born by caesarean section (Fig. 1).

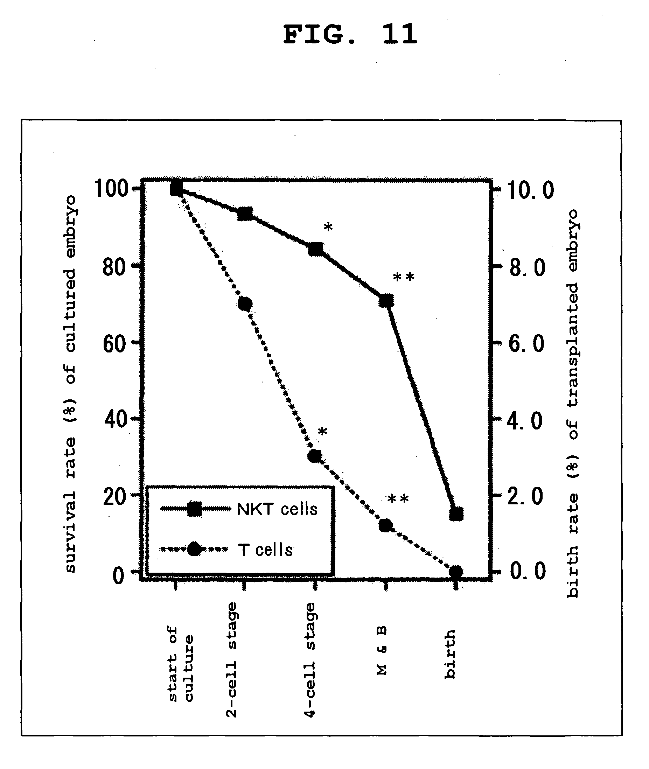

272 embryos were transplanted in a pseudopregnant mouse and four babies were born (probability about 1.5%). In addition, 13 conception products of placenta alone were obtained (probability about 4.8%). These results are extremely similar to those using ES cell as a donor, and it as found that NKT cell, which is a differentiated somatic cell, was effective for reprogramming of genome for the reproduction of mouse, like ES cell. The cloned placenta all showed moderate or high level of placenta hyperplasia observed in mouse somatic cell cloning. The above-mentioned results are summarized in Table 1 and Fig. 11.

**Table 1**

| cell type | NKT cell | | T cell | |
|---|---|---|---|---|
| | 48 h | 72 h | 48 h | 72 h |
| number of cultured cells | 280 | 292 | 105 | 232 |
| number (%) of cells in 2-cell stage | 260 (93) | 274 (94) | 62 (59) | 174 (75) |
| number (%) of cells in 4-cell stage or later | 241 (86) | 241 (83) | 21 (20) | 81 (35) |
| number (%) of M&B cells | | 207 (71) | | 28 (12) |
| number (%) of transplanted embryos | 185 | 87 | 21 | 23 |
| number (%) of implantation | 112 (61) | 49 (56) | 3 (14) | 0 (0) |
| number (%) of fetus | 3 (1.6) | 1 (1.1) | 0 (0) | 0 (0) |
| number (%) of placenta alone | 5 (2.7) | 8 (9.2) | 0 (0) | 0 (0) |

| | | | | |
|---|---|---|---|---|
| M&B: morula and blastocyst | | | | |

To verify that the obtained cloned mouse is of NKT cell origin, Vα14 which is a subunit of TCR unique to the cell was detected by Southern blot.

NKT cell has a TCRVα chain consisting of a combination of Vα14-Jα281 alone. If the prepared cloned mouse is derived from NKT cell, the genomic DNA of the mouse, and the placenta should have a genomic DNA of Vα14-Jα281 after gene rearrangement. Similarly, Vβ chain should have a genomic DNA after gene rearrangement. The probe for Southern blot was prepared as follows.

### Preparation of probe for Southern blot

Using the following primer set and genomic DNA extracted from the tail of C57BL/6 mouse as a template, PCR reaction was performed. Each primer was prepared utilizing the custom primer synthesis of In Vitrogen.

The base sequence of the PCR product was confirmed by DNA sequencing.
<PCR primer for preparation of TCRV_{α}14 Southern probe> primer sequence 1: 5'-CGCTTGTGCACATTTGTTCT-3' (SEQ ID NO: 1)
primer sequence 2: 5'-TAAGTTTCTGGGGAGCATGG-3' (SEQ ID NO: 2)
<PCR primer for preparation of TCRVβ Southern probe> primer sequence 3: 5'-GGGGCTGTGAACCAAGACAC-3' (SEQ ID NO: 3)
primer sequence 4: 5'-TACTCATTTCGCTCCTTTCAAAAGACC-3' (SEQ ID NO: 4)

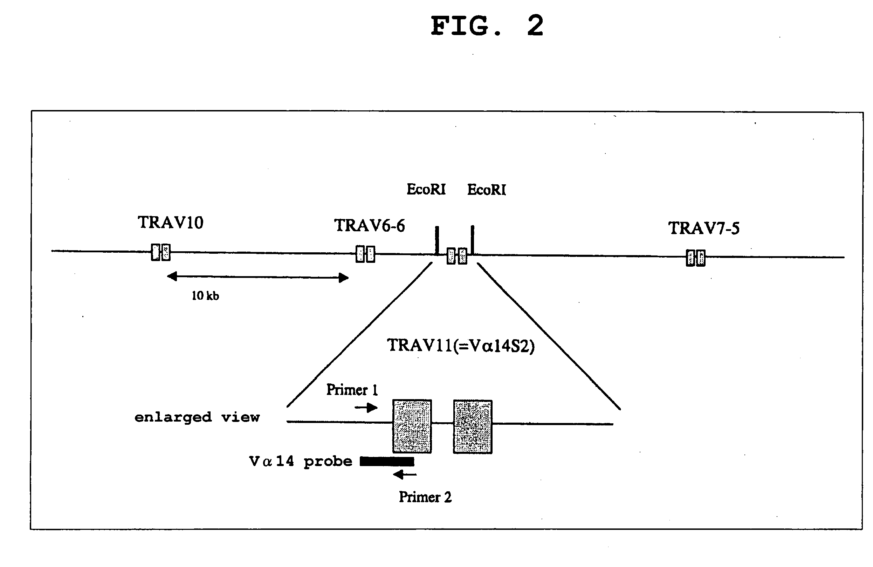

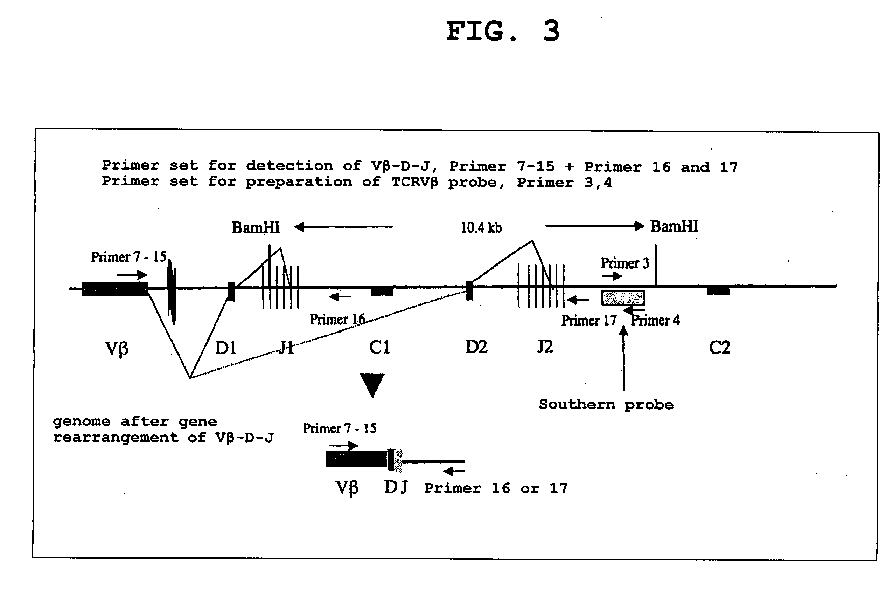

Fig. 2 shows the position of TCRVα14 Southern probe on the genome, and Fig. 3 shows the position of TCRVβ Southern probe on the genome. Fig. 2 schematically shows various Vα fragments in the vicinity of Vα14 gene locus. The exon moiety is shown with squares. Fig. 3 schematically shows respective Vβ, D, J and C segments.

The genomic DNA (5-30 µg) was digested with EcoRI (for TCRVα14) or BamHI (for TCRVβ), and electrophoresed to separate DNA fragments, which were transferred onto a nylon membrane Hybond N+ (trade name, Amersham). After UV crosslinking, the membrane was prehybridized in a PerfectHyb (registered name, TOYOBO) solution (68°C, 60 min), TCRVα14 Southern probe or TCRVβ Southern probe RI-labeled with Rediprime II (Amersham) was added, and hybridization was performed at 68°C for 16 hr. Thereafter, the membrane was washed twice with 2xSSC, 0.1% SDS (68°C) solution for 5 min, and additional twice with 0.1xSSC, 0.1% SDS (68°C) solution for 15 min. The nylon membrane after washing was exposed on Image plate (Fuji Film), and analyzed using Image plate Reader BAS 2500 (Fuji Film).

Genetic rearrangement of Vα14 chain was observed in all mice that were born. The results are shown in Fig. 4.

Since genomic DNA having a germline configuration, which is free of gene rearrangement, does not have a rearranged product (Vα-Jα), Southern blot using a Vα14 probe gives rise to in a 2.5 kb band in a case having a 129/Sv-ter mouse-derived allele (Fig. 4, lane 2), or an about 13kb band in a case having a C57BL/6 mouse-derived allele (Fig. 4, lane 1). This is because fragments resulting from digestion with EcoRI differ between 129/Sv-ter origin and C57BL/6 origin. C57BL/6 x 129/Sv-ter mouse, from which the donor cell derives, has both alleles, giving both the about 2.5kb band and the about 13kb band (Fig. 4, lane 3). In cloned mice #1, #3 and #4, an 8 kb band became detectable by recombination but about 13 kb and 2.5 kb bands remained intact, in the genomic DNA having a Vα14 gene locus after gene rearrangement (Fig. 4, lane 4 and lane 5, lane 8, lane 9). Combined with the results of base sequence determination of the TCRVα chain mentioned below, it is clear that the 8 kb band is derived from a C57BL/6-derived allele in a cloned mouse. The reason for the remaining of the about 13 kb band even after gene rearrangement is considered to be that the C57BL/6 allele has two Vα14 genes and one of them is a pseudogene.

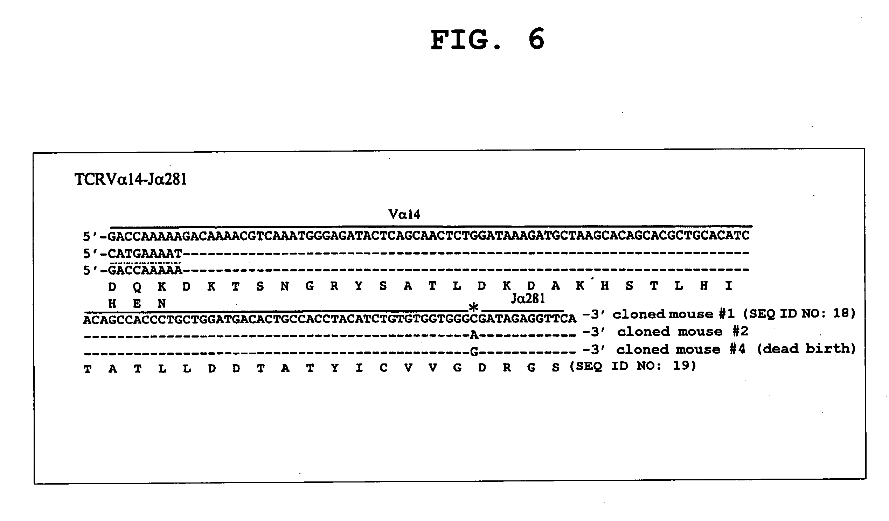

In cloned mouse #2, moreover, an 8 kb band became detectable by recombination but an about 13 kb band remained intact and a 2.5 kb band disappeared, in the genomic DNA having a Vα14 gene locus after gene rearrangement. Therefrom it is assumed that gene rearrangement occurred in 129/Sv-ter-derived allele as well (Fig. 4, lane 6 and lane 7). From the results of DNA base sequence (Fig. 6), it has been clarified that 129/Sv-ter-derived Vα14-Jα281 is used in cloned mouse #2.

Southern blot of TCRVβ in the same manner revealed rearrangement of allele on one or both sides. While genomic DNA of germline configuration free of gene rearrangement shows only a 10.4 kb band, genomic DNA having a gene locus after gene rearrangement shows various sizes of bands by recombination. The results are shown in Fig. 4.

In addition, an empty placenta without a baby may be obtained during preparation of a cloned mouse. Genomic DNA of the obtained empty placenta was extracted and subjected to Southern blot analysis of TCRVα14 and TCRVβ in the same manner. As a result, the Southern blot pattern of the empty placenta-derived genomic DNA matched with the Southern blot pattern of the cloned mouse tail-derived genomic DNA in the Vα14 chain and Vβ chain. (In a placenta-derived genomic DNA, a band derived from a foster mother may be observed. 10.4 kb for TCRVβ).

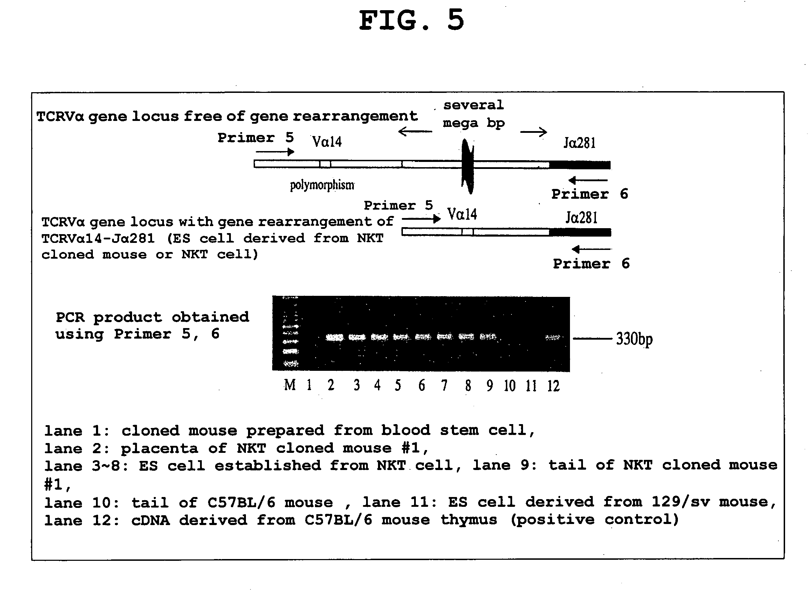

Using these genomic DNAs as templates, PCR was performed and the DNA sequences of the Vα chain and Jα chain were examined. Genomic DNA was extracted from the tail or placenta of the cloned mice (cloned mice #1, #2 and #4) and the sequence of Vα14-Jα281 was amplified by PCR. When both alleles of the genomic DNA are both of the configuration before gene rearrangement (germline configuration), a PCR product does not occur. This is because the gene segments of Vα14 and Jα281 are present several mega bases or above away from each other, and cannot be amplified by PCR. Once genetically rearranged, however, an about 330 bp PCR product should be afforded (see Fig. 5). Furthermore, since Vα14 gene has a sequence polymorphism, once the DNA sequence of the obtained product is determined, whether its Vα is derived from 129/Sv-ter or C57BL/6 can be known (donor NKT cell is F1 of C57BL/6 and 129/Sv-ter) (see Fig. 6).

PCR primer for detection of Vα14-Jα281 was as follows. Fig. 5 schematically shows the position of each primer on the genome.
<PCR primer for detection of Vα14-Jα281>
primer sequence 5: 5'-CCCAAGTGGAGCAGAGTCCT-3' (SEQ ID NO: 5)
primer sequence 6: 5'-AGGTATGACAATCAGCTGAGTCC-3' (SEQ ID NO: 6)

Using genomic DNA from the placenta and tail of a cloned mouse as a template and a combination of the above-mentioned primers (primers 5 and 6), PCR was performed (as 50 ng of template DNA using AmpliTaq Gold of ABI at primer concentration of 0.2 µM, treatment at 94°C for 10 min and 36 cycles of 94°C 1 min, 60°C 1 min, 72°C 1 min as one cycle were performed). As a result, an about 330 bp PCR product was obtained (Fig. 5, PCR, lane 2 and lane 9, respectively). On the other hand, genomic DNA of C57BL/6, 129/Svj or blood stem cell-derived cloned mouse, which was a control, failed as expected to give a product (Fig. 5). DNA base sequence of the PCR product was determined. As shown in Fig. 6, the PCR product was found to have TCRVα14-Jα281 in-frame after gene rearrangement. The cloned mice #1 and #4 were of C57BL/6 type, and the cloned mouse #2 was of 129/Sv-ter type.

In NKT cell, the TCRVβ chain is not unambiguously determined unlike TCRVα chain. To examine which Vβ chain is used, PCR was performed using a primer capable of detecting various Vβ chains and tail and placental genomic DNA of cloned mouse #1 as a template, and DNA base sequence was determined (see Fig. 3 for the position of each primer on the genome). The combination of the PCR primers used was any one primer 5 from the following primer group A and any one primer from the primer group B. Also in this case, like TCRV_{α} chain, a PCR product can be obtained from the allele having a TCRVβ gene locus after gene rearrangement, but a normal allele having a germline configuration does not afford a product.
<PCR primer for detection of TCRVβ>
primer group A (TCRVβ side);
primer sequence 7 (for Vβ2) : 5'-CACGGGTCACTGATACGGAGC-3' (SEQ ID NO: 7)
primer sequence 8 (for Vβ3): 5'-TGAGTGTCCTTCAAACTCACC-3' (SEQ ID NO: 8)
primer sequence 9 (for Vβ4): 5'-AAACCATTTAGACCTTCAGAT-3' (SEQ ID NO: 9)
primer sequence 10 (for Vβ5): 5'-AGTTTGATGACTATCACTCTG-3' (SEQ ID NO: 10)
primer sequence 11 (for Vβ6): 5'-GGGCAAAAACTGACCTTGAA-3' (SEQ ID NO: 11)
primer sequence 12 (for Vβ7): 5'-TCTCACGGAAGAAGCGGGAGC-3' (SEQ ID NO: 12)
primer sequence 13 (for Vβ8): 5'-GATACAAGGCCTCCAGACCA-3' (SEQ ID NO: 13)
primer sequence 14 (for Vβ11): 5'-GCCCAATCAGTCGCACTCAAC-3' (SEQ ID NO: 14)
primer sequence 15 (for Vβ12): 5'-CATCCTTCTCCACTCTGAAGA-3' (SEQ ID NO: 15) primer group B;
primer sequence 16: 5'-GAAGGGACGACTCTGTCTTACCTT-3' (SEQ ID NO: 16)
primer sequence 17: 5'-TGAGAGCTGTCTCCTACTATCGATT-3' (SEQ ID NO: 17)

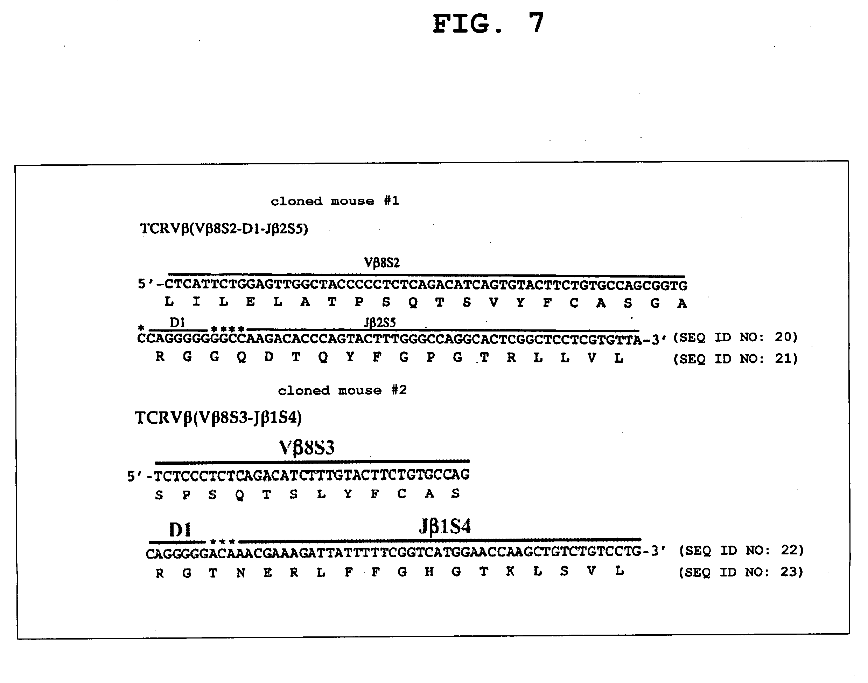

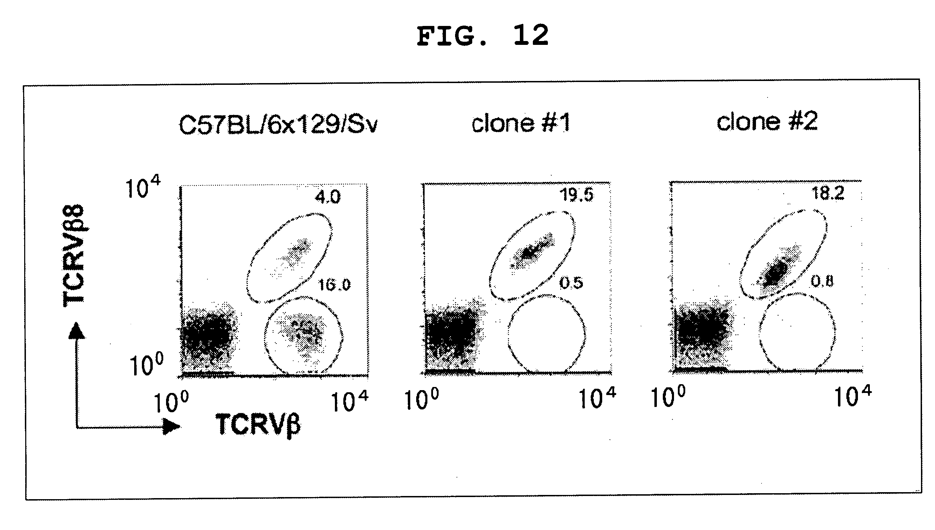

The results of the determined base sequence are shown in Fig. 7. The cloned mouse #1 had a frame of in-frame Vβ8S2-D1-Jβ2S5 after gene rearrangement and that of cloned mouse #2 was Vβ8S3-D1-Jβ1S4. Moreover, the peripheral blood of the cloned mice #1 and #2 was analyzed for Vβ phenotype by FACS. As a result, the phenotype was different from that of the donor cell, and was mostly TCRVβ8 positive (Fig. 12). This indicates incident of allelic exclusion in both cloned mice. These cloned mice do not show any apparent abnormality and are completely normal except a slight tendency of overweight at the present stage of 12 months or longer after the birth (as of June 2005).

From the above results, it has been confirmed that the cloned mouse obtained in the present invention is derived from peripheral NKT cell after gene rearrangement.

### Example 2: Establishment of ES cell line from NKT cell

Using the nucleus derived from NKT cell, establishment of ES cell was tried by the direct nuclear transplantation method described in Example 1. 97 NKT cells were damaged with a pipette and transplanted in the oocyte denucleated by the aforementioned method. At 60 hr after transplantation (Day 2.5, 8-cell stage, embryo), the cells were washed with a medium for ES (DMEM containing FCS, L-glu, NEAA, P/S, LIF and 2ME), and inoculated onto fetal fibroblast prepared in advance (1 embryo/culture dish). The cells were cultured at 37°C, 7%CO₂ up to the sufficient growth of inner cell mass (ICM) (about 7-10 days). As a result, cells containing 16 ICMs were obtained. ICM was divided into small pieces with a syringe and a needle, and the culture was continued until an ES colony was confirmed. As a result of these series of steps, 11 ES cell lines were obtained. Of these, 5 were differentiated, but 6 ES cell lines were established. The chimeric mouse forming ability of these 6 ES cell lines was examined. As a result, 4 ES cells were confirmed to have an ability to form a chimeric mouse. The series of the progresses are summarized in Table 2.

**Table 2**

| ES cells | |
|---|---|
| Number of NKT cells used for nuclear transplantation | 97 |
| Number of blastocysts that formed ICM | 16 |
| Number of ES cell lines established from ICM (including differentiated cell lines) | 11 |
| Number of ES cells established while undifferentiated | 6 |
| Number of ES cell lines confirmed to have chimeric mouse forming ability | 4 |

| | |
|---|---|
| ES cell line establishing rate (6/97), 6% | |

The obtained ES cells were analyzed by PCR for the TCRVα gene locus in the same manner as in Example 1 (Fig. 5, lanes 3 - 8). As a result, all of them showed 330 bp PCR product, whereby the incident of gene rearrangement was verified. Moreover, Southern blot analysis was performed in the same manner as in Example 1. As a result, they were confirmed to have a genetically rearranged TCRVα14 chain, which is the same as in the donor NKT cell, and the TCRVβ chain was also confirmed to have been genetically rearranged (Fig. 8, lanes 1 - 6).

### Example 3: Offspring of cloned mouse (reproductive ability of cloned mouse)

To examine the reproductive ability of a NKT cell-derived cloned mouse, cloned mouse #1 was crossed with ICR, C57BL/6 female mouse. As a result, 47 mice were born in 3 months. Based on this fact, the cloned mouse was considered to have normal reproductive ability. Genomic DNA of these children (offspring F1) was confirmed by Southern blot analysis. It was confirmed that male No. 1 and female No. 6 were F1 that inherited 8 kb band derived from cloned mouse-derived TCRVα14 after gene rearrangement, and allele TCRVα14 chain of the cloned mouse after gene rearrangement was inherited by a germline cell (Fig. 9, upper panel, arrows). In addition, the allele containing parent cloned mouse #1-derived TCRVβ was separated into about 9 kb and 8 kb (see Fig. 4), and each F1 inherited a mother mouse-derived 10.4 kb band (Fig. 9, lower panel, open arrow heads) and an about 9 kb or 8 kb band (Fig. 9, lower panel, closed arrow heads). For example, it is clear male No. 1, and female Nos. 3, 6 and 7 inherited about 9 kb band, and male Nos. 2 - 6, female Nos. 1, 2, 4, 5 and 8 inherited about 8 kb band. In other words, it was clarified that one allele containing a genome after gene rearrangement of the father cloned mouse is inherited in the TCRVβ chain (Fig. 9). From the foregoing facts, it was clarified that the genomic DNA after gene rearrangement was transmitted to an offspring.

The cell surface antigens of T cell and NKT cell of the offspring of NKT cloned mouse having an allele after gene rearrangement were analyzed by FACS. The spleen and liver of NKT cloned mouse F1 were analyzed. The spleen was crushed on two pieces of slide glass into single cell, erythrocytes were removed and the rest was used for staining. The liver was crushed with a metal mesh having a 75 µm diameter, prepared to mononuclear cells by the density gradient of Percoll, after which erythrocytes were removed and the single cells were used for staining. These cells were stained with anti-TCRVβ antibody (same as in Example 1) that recognizes whole TCRVβ and antibody that recognizes only TCRVβ8 (PharMingen, product No. 553861) and analyzed by FACS, based on which the proportion of TCRVβ8 in the offspring of cloned mouse was examined. The results are shown in the upper panel of Fig. 10. Similarly, the spleen and liver were degraded to single cells, stained with CD1d tetramer (same as in Example 1) and anti-TCRVβ antibody (same as in Example 1) and analyzed by FACS, based on which the proportion of NKT cell in the offspring of cloned mouse was examined. The results are shown in the lower panel of Fig. 10. The rate of TCRVβ8 expression in the spleen was a little over 20% of T cell in wild-type but nearly 100% in the mouse that inherited in-frame TCRVβ8 (Fig. 10, panel 2). The same tendency was observed in the liver. In the wild-type spleen cells, the rate of NKT cells as defined by α-GC loaded CD1d-tetramer⁺/TCRβ⁺ was about 0.5% of the whole spleen cells, but the rate increased to about 13% in the mouse that inherited V_{α}14-J_{α}281 after gene rearrangement (Fig. 10, panel 3). In this mouse, the total number of spleen cells was about half that of the wild-type but the absolute number of NKT cells was about 13-fold (Fig. 10).

From the foregoing results, in a cloned mouse offspring that genetically inherited in-frame TCRVβ chain after gene rearrangement, the TCRVβ chain expressed on its T cell consists of unambiguous Vβ chain repertoire almost entirely defined in-frame. That is, allelic exclusion observed in a transgenic mouse is observed. Moreover, an offspring that inherited TCRV_{α}14-J_{α}281 shows 10- to 20-fold increased absolute number and rate of NKT cells, as compared to the wild-type. While these results concerns F1 obtained using ICR, similar tendencies were observed in F1 obtained by crossing with C57BL/6.

### Example 4: Offspring of cloned mouse (function of NKT cells)

In this Example, the function of NKT cells of an offspring of a cloned mouse was confirmed. Cytokine production by stimulation with α-galactosylceramide (α-GC), which is an artificial agonist of NKT cell, was examined in vitro and in vivo.

Cloned mouse #1 and female C57BL/6 were crossed to prepare F1. Thereafter, male F1 containing Vα14-Jα281 after gene rearrangement were to be SPF by IVF (in vitro fertilization), and crossed again with female C57BL/6 to give an offspring (Vα14-Jα281 mouse). Three-month-old V_{α}14-J_{α}281 mouse and a littermate of the same age (control mouse) were used for the experiment.

### Cytokine production by in vivo stimulation (method)

_{α}-GC was intraperitoneally administered to a mouse at a dose of 2 µg/mouse for in vivo stimulation. The serum of the mouse after in vivo stimulation was taken at a given time, and various cytokine concentrations in the serum were measured by the Bio-Plex Suspension Array System (BioRad, Hercules, CA).

### (results)

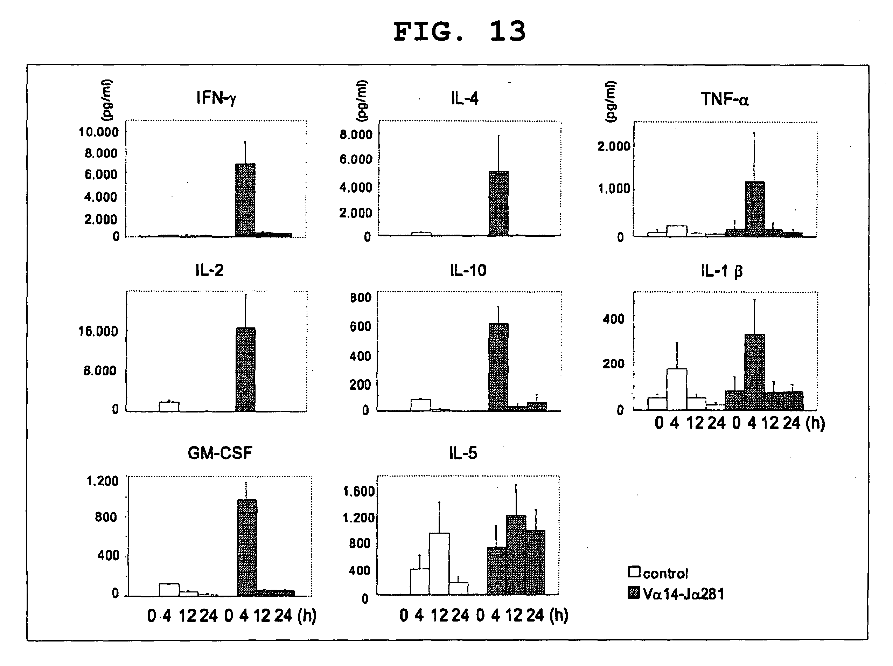

The results are shown in Fig. 13. In V_{α}14-J_{α}281 mouse (mouse hetero inherited V_{α}14-J_{α}281 after gene rearrangement in the germline), enhancement in the cytokine (IL (interleukin)-2,4,10, GM-CSF (granulocyte macrophage colony stimulating factors), IL-1β, TNFα (tumor necrosis factor α) , IFN (interferon)-γ)-producing ability was confirmed. In addition to the increased absolute number and rate of NKT cells as confirmed in Example 3, maintenance of its function was also confirmed.

### IL-4 and IFN-γ production by in vitro stimulation (method)

Using CD11c microbeads (Miltenyi), dendritic cells (DC) were obtained from the spleen of mouse (Vα14-Jα281 mouse, or littermate control mouse). The dendritic cells derived from each mouse was stimulated with α-GC (200 ng/mL) for 6 hr, and mixed with the spleen cells (SPC) derived from each mouse. These cells were co-cultured for 72 hr and the production of IFN-γ and IL-4 was measured using an ELISA kit (Genzyme TECHNE, Meneapolis, MN).

### (results)

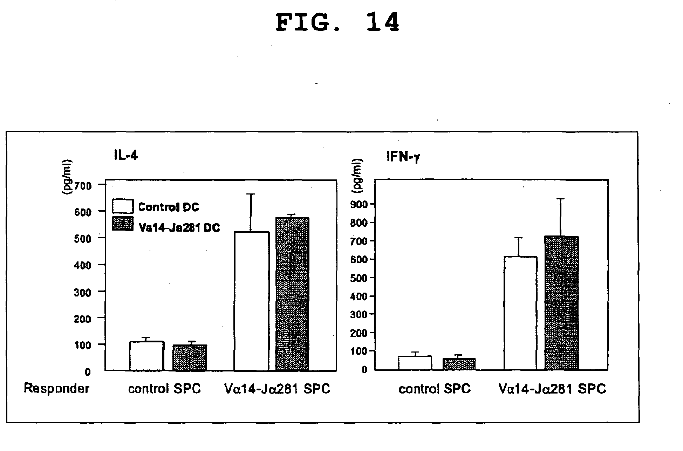

The results are shown in Fig. 14. It was confirmed that IL-4 and IFN-γ-producing ability was enhanced in the V_{α}14-J_{α}281 mouse-derived spleen cells, as compared to the control mouse.

### SEQUENCE LISTING FREE TEXT

SEQ ID NO: 1: PCR primer for preparation of Southern probe of TCRV_{α}14 (primer sequence 1)
SEQ ID NO: 2: PCR primer for preparation of Southern probe of TCRV_{α}14 (primer sequence 2)
SEQ ID NO: 3: PCR primer for preparation of Southern probe of TCRVR (primer sequence 3)
SEQ ID NO: 4: PCR primer for preparation of Southern probe of TCRVβ (primer sequence 4)
SEQ ID NO: 5: PCR primer for detection of TCRV_{α}14-J_{α}281 (primer sequence 5)
SEQ ID NO: 6: PCR primer for detection of TCRVα14-Jα281 (primer sequence 6)
SEQ ID NO: 7: PCR primer for detection of TCRVβ2 (primer sequence 7)
SEQ ID NO: 8: PCR primer for detection of TCRVβ3 (primer sequence 8)
SEQ ID NO: 9: PCR primer for detection of TCRVβ4 (primer sequence 9)
SEQ ID NO: 10: PCR primer for detection of TCRVβ5 (primer sequence 10)
SEQ ID NO: 11: PCR primer for detection of TCRVβ6 (primer sequence 11)
SEQ ID NO: 12: PCR primer for detection of TCRVβ7 (primer sequence 12)
SEQ ID NO: 13: PCR primer for detection of TCRVβ8 (primer sequence 13)
SEQ ID NO: 14: PCR primer for detection of TCRVβ11 (primer sequence 14)
SEQ ID NO: 15: PCR primer for detection of TCRVβ12 (primer sequence 15)
SEQ ID NO: 16: PCR primer for detection of TCRVβ (primer sequence 16)
SEQ ID NO: 17: PCR primer for detection of TCRV_{β} (primer sequence 17)

### Industrial Applicability

The cloned mammal and cloned mammalian embryo obtained by the present invention, as well as the cells, tissues, organs and products obtained therefrom have exactly the same tissue compatible antigen (e.g., HLA), and show equivalent antigenicity, and therefore, they are free of immunorejection during transplantation and administration. In addition, when NKT cell is used, preparation of clone does not require establishment of ES cell and tetraploid complementation. As a result, drastic saving of the time and labor can be achieved. Moreover, when ES cell is established using the nucleus of NKT cell, its efficiency is about 6%, which is considerably higher than by the use of conventional peripheral mouse T cell or B cell as a donor. It is considerably higher than the efficiency of establishment of human ES cell by autologous transplantation (Hwang, W.S. et al., "Evidence of a pluripotent human embryonic stem cell line derived from a cloned blastocyst", Science, (US), 2004, 303, p. 1669-1674). Furthermore, the rate of growing into an individual from the transplanted nucleus is about 1.5%, which is comparable to the success rate in creating a clone from ES cell, which is considered to produce an individual comparatively easily (Wakayama, T. et al., "Mice cloned from embryonic stem cells", Proceedings of the National Academy of Sciences of the United States of America, (US), 1999, 96, p. 14984-14989).

Using the method of the present invention, a cloned mammal can be produced at a higher rate. Therefore, useful animals (e.g., transgenic sheep, cow etc. that produce bio-pharmaceutical and the like in milk) can be cloned easily, which in turn enables mass production and cost reduction of such pharmaceutical products, thus contributing to the reduction of total medical expense.

Furthermore, the offspring of the cloned mammal of the present invention is characterized in that the TCRVβ chain expressed on T cell consists of a substantially single TCRVβ chain repertoire. More particularly, the TCRVβ chain repertoire is the same as that of the NKT cell used as a donor cell. Moreover, an offspring having an allele containing V_{α}14-J_{α}281, which is a TCR_{α} chain after gene rearrangement, has a relatively and absolutely increased number of NKT cells as compared to that of a wild-type. From the foregoing facts, an offspring of the cloned mammal of the present invention gives, as a model animal of pathologies such as autoimmune diseases and the like, a means for analyzing the role of NKT cell in immune control and significance afforded by the variety of TCRVβ chain repertoires.

5 This application is based on application Nos. 2004-238836 and 2005-177998 filed in Japan, the contents of which are incorporated hereinto by reference.

## Claims

1. A method for preparing a cloned non-human mammal, which comprises using a mammalian natural killer T cell as the donor cell.

2. A method for preparing a cloned non-human mammal, which comprises introducing the nucleus of a mammalian natural killer T cell into an enucleated mammalian oocyte.

3. A method for preparing a cloned non-human mammal, which comprises introducing a nucleus derived from a mammalian natural killer T cell into an enucleated mammalian oocyte to form a reconstructed embryo, and transferring the reconstructed embryo into a host mammal.

4. The method for preparing a cloned non-human mammal of any one of claims 1 to 3, wherein the natural killer T cell has been genetically manipulated to express a desired character.

5. The method for preparing a cloned non-human mammal of any one of claims 1 to 4, wherein the natural killer T cell and the enucleated oocyte are derived from the same species of mammal.

6. The method for preparing a cloned non-human mammal of any one of claims 1 to 4, wherein the natural killer T cell and the enucleated oocyte are derived from different species of mammals.

7. The method for preparing a cloned non-human mammal of any one of claims 1 to 6, wherein the natural killer T cell is collected from a tissue selected from the group consisting of cord blood, peripheral blood, liver, bone marrow, spleen, and thymus.

8. A cloned non-human mammal prepared by the method of any one of claims 1 to 7.

9. A fetus of a cloned non-human mammal obtained by the method of any one of claims 1 to 7.

10. An offspring of the cloned non-human mammal of claim 8.

11. The offspring of claim 10, wherein the TCRVβ chain expressed on T cells is constituted by a substantially single TCRVβ chain repertoire.

12. The offspring of claim 11, wherein the single TCRVβ chain repertoire is identical to the TCRVβ chain repertoire of the natural killer T cell used as the donor cell.

13. The offspring of claim 10, which has an allele comprising V_{α}14-J_{α}281, which is a TCR_{α} chain after gene rearrangement, wherein the number of natural killer T cells has been increased.

14. A cell, tissue, organ or product obtained from the cloned non-human mammal of claim 8.

15. A cell, tissue, organ or product obtained from the fetus of a cloned non-human mammal of claim 9.

16. A cell, tissue, organ or product obtained from the offspring of cloned non-human mammal of claim 10.

17. The cell, tissue, organ or product of claim 14 or 15, which is immunologically identical to the donor cell.

18. The cell, tissue, organ or product of any one of claims 14 to 17, which is used for a treatment, an organ transplantation and/or a cell transplantation.

19. A method for preparing a cloned mammal embryo, which comprises using a mammalian natural killer T cell as a donor cell.

20. A method for preparing a cloned mammal embryo, which comprises introducing the nucleus of a mammalian natural killer T cell into an enucleated mammalian oocyte.

21. The method of claim 19 or 20, wherein the natural killer T cell has been genetically manipulated to express a desired character.

22. The method of any one of claims 19 to 21, wherein the natural killer T cell and the enucleated oocyte are derived from the same species of mammal.

23. The method of any one of claims 19 to 21, wherein the natural killer T cell and the enucleated oocyte are derived from different species of mammals.

24. The method of any one of claims 19 to 23, wherein the natural killer T cell is collected from a tissue selected from the group consisting of cord blood, peripheral blood, liver, bone marrow, spleen, and thymus.

25. A cloned mammal embryo prepared by the method of any one of claims 19 to 24.

26. A method for preparing a cloned mammal embryonic stem cell, which comprises culturing the cloned mammal embryo of claim 25 up to the blastocyst stage or the pre-blastocyst stage, and separating an embryonic stem cell from the obtained inner cell mass in the blastocyst stage or the pre-blastocyst stage.

27. A cloned mammal embryonic stem cell obtained by the method of claim 26.

28. A method for preparing a differentiated cell, tissue, organ or product of a cloned mammal, which comprises culturing the cloned mammal embryonic stem cell of claim 27 under conditions allowing the induction of desired cell differentiation to induce the differentiation.

29. A differentiated cell, tissue, organ or product of a cloned mammal obtained by the method of claim 28.

30. The differentiated cell, tissue, organ or product of claim 29, which is immunologically identical to the donor cell.

31. The differentiated cell, tissue, organ or product of claim 29 or 30, which is used for a treatment, an organ transplantation and/or a cell transplantation.


SEQUENCE LISTING

<110> RIKEN

<120> Method for Producing a Cloned Mammal

<130> 09794

<150> JP 2004-238836
<151> 2004-8-18

<150> JP 2005-177998
<151> 2005-6-17

<160> 23

<170> PatentIn version 3.1

<210> 1
<211> 20
<212> DNA
<213> Artificial sequence

<220>

<223> PCR primer for preparation of Southern blot probe of TCRV alpha 14

<400> 1
cgcttgtgca catttgttct                                                    20

<210> 2
<211> 20
<212> DNA
<213> Artificial sequence

<220>

<223>  PCR primer for preparation of Southern blot probe of TCRV alpha 14

<400>  2
taagtttctg gggagcatgg                                                    20

<210>  3
<211>  20
<212>  DNA
<213>  Artificial sequence

<220>

<223>  PCR primer for preparation of Southern blot probe of TCRV beta

<400>  3
ggggctgtga accaagacac                                                    20

<210>  4
<211>  27
<212>  DNA
<213>  Artificial sequence

<220>

<223>  PCR primer for preparation of Southern blot probe of TCRV beta

<400>  4
tactcatttc gctcctttca aaagacc                                            27

<210>  5
<211>  20
<212>  DNA

<213>  Artificial sequence

<220>

<223>  PCR primer for detection of TCRV alpha 14-J alpha 281

<400>  5
cccaagtgga gcagagtcct                                                    20

<210>  6
<211>  23
<212>  DNA
<213>  Artificial sequence

<220>

<223>  PCR primer for detection of TCRV alpha 14-J alpha 281

<400>  6
aggtatgaca atcagctgag tcc                                                23

<210>  7
<211>  21
<212>  DNA
<213>  Artificial sequence

<220>

<223>  primer for TCRV beta 2

<400>  7
cacgggtcac tgatacggag c                                                  21

<210>  8
<211>  21

<212>  DNA
<213>  Artificial sequence

<220>

<223>  primer for TCRV beta 3

<400>  8
tgagtgtcct tcaaactcac c                                                21

<210>  9
<211>  21
<212>  DNA
<213>  Artificial sequence

<220>

<223>  primer for TCRV beta 4

<400>  9
aaaccattta gaccttcaga t                                                21

<210>  10
<211>  21
<212>  DNA
<213>  Artificial sequence

<220>

<223>  primer for TCRV beta 5

<400>  10
agtttgatga ctatcactct g                                                21

<210>  11

<211>  20
<212>  DNA

<213>  Artificial sequence

<220>

<223>  primer for TCRV beta 6

<400>  11
gggcaaaaac tgaccttgaa                                                    20

<210>  12
<211>  21
<212>  DNA

<213>  artificial sequence

<220>

<223>  primer for TCRV beta 7

<400>  12
tctcacggaa gaagcgggag c                                                  21

<210>  13
<211>  20
<212>  DNA
<213>  Artificial sequence

<220>

<223>  primer for TCRV beta 8

<400>  13

gatacaaggc ctccagacca 20

<210> 14
<211> 21
<212> DNA
<213> Artificial sequence

<220>

<223> primer for TCRV beta 11

<400> 14
gcccaatcag tcgcactcaa c 21

<210> 15
<211> 21
<212> DNA
<213> Artificial sequence

<220>

<223> primer for TCRV beta 12

<400> 15
catccttctc cactctgaag a 21

<210> 16
<211> 24
<212> DNA
<213> Artificial sequence

<220>

<223> primer for TCRV beta

<400> 16
gaagggacga ctctgtctta cctt 24

<210> 17
<211> 25
<212> DNA
<213> Artificial sequence

<220>

<223> primer for TCRV beta

<400> 17
tgagagctgt ctcctactat cgatt 25

<210> 18
<211> 138
<212> DNA
<213> Mus musculus

<220>
<221> CDS
<222> (1)..(138)
<223>

<400> 18
gac caa aaa gac aaa acg tca aat ggg aga tac tca gca act ctg gat 48
Asp Gln Lys Asp Lys Thr Ser Asn Gly Arg Tyr Ser Ala Thr Leu Asp
1               5                   10                  15

aaa gat gct aag cac agc acg ctg cac atc aca gcc acc ctg ctg gat 96
Lys Asp Ala Lys His Ser Thr Leu His Ile Thr Ala Thr Leu Leu Asp
            20                  25                  30

gac act gcc acc tac atc tgt gtg gtg ggc gat aga ggt tca 138

Asp Thr Ala Thr Tyr Ile Cys Val Val Gly Asp Arg Gly Ser
        35                  40                  45

<210>  19
<211>  46
<212>  PRT
<213>  Mus musculus

<400>  19

Asp Gln Lys Asp Lys Thr Ser Asn Gly Arg Tyr Ser Ala Thr Leu Asp
1               5                   10                  15

Lys Asp Ala Lys His Ser Thr Leu His Ile Thr Ala Thr Leu Leu Asp
            20                  25                  30

Asp Thr Ala Thr Tyr Ile Cys Val Val Gly Asp Arg Gly Ser
        35                  40                  45

<210>  20
<211>  114
<212>  DNA
<213>  Mus musculus

<220>
<221>  CDS
<222>  (1)..(114)
<223>

<400>  20
ctc att ctg gag ttg gct acc ccc tct cag aca tca gtg tac ttc tgt       48
Leu Ile Leu Glu Leu Ala Thr Pro Ser Gln Thr Ser Val Tyr Phe Cys
1               5                   10                  15

gcc agc ggt gcc agg ggg ggc caa gac acc cag tac ttt ggg cca ggc       96

Ala Ser Gly Ala Arg Gly Gly Gln Asp Thr Gln Tyr Phe Gly Pro Gly
            20                  25                  30

act cgg ctc ctc gtg tta 114
Thr Arg Leu Leu Val Leu
        35

<210> 21
<211> 38
<212> PRT
<213> Mus musculus

<400> 21

Leu Ile Leu Glu Leu Ala Thr Pro Ser Gln Thr Ser Val Tyr Phe Cys
1               5                   10                  15

Ala Ser Gly Ala Arg Gly Gly Gln Asp Thr Gln Tyr Phe Gly Pro Gly
            20                  25                  30

Thr Arg Leu Leu Val Leu
        35

<210> 22
<211> 90
<212> DNA
<213> Mus musculus

<220>
<221> CDS
<222> (1)..(90)

<400> 22
tct ccc tct cag aca tct ttg tac ttc tgt gcc agc agg ggg aca aac 48
Ser Pro Ser Gln Thr Ser Leu Tyr Phe Cys Ala Ser Arg Gly Thr Asn

1                5                   10                  15

gaa aga tta ttt ttc ggt cat gga acc aag ctg tct gtc ctg              90
Glu Arg Leu Phe Phe Gly His Gly Thr Lys Leu Ser Val Leu
            20                  25                  30

<210>  23
<211>  30
<212>  PRT
<213>  Mus musculus

<400>  23

Ser Pro Ser Gln Thr Ser Leu Tyr Phe Cys Ala Ser Arg Gly Thr Asn
1               5                   10                  15

Glu Arg Leu Phe Phe Gly His Gly Thr Lys Leu Ser Val Leu
            20                  25                  30